(19) Europäisches Patentamt
European Patent Office
Office européen des brevets

(11) Veröffentlichungsnummer: **0 279 458 A1**

(12) **EUROPÄISCHE PATENTANMELDUNG**

(21) Anmeldenummer: **88102467.3**

(22) Anmeldetag: **19.02.88**

(51) Int. Cl.⁴: **C07C 93/16**, C07C 93/04, C07C 149/24, A61K 31/215

Patentansprüche für folgende Vertragsstaaten: GR + ES.

(30) Priorität: **20.02.87 DE 3705384**

(43) Veröffentlichungstag der Anmeldung: **24.08.88 Patentblatt 88/34**

(84) Benannte Vertragsstaaten: **AT BE CH DE ES FR GB GR IT LI LU NL SE**

(71) Anmelder: **GÖDECKE AKTIENGESELLSCHAFT**
**Salzufer 16**
**D-1000 Berlin 10(DE)**

(72) Erfinder: **Trostmann, Uwe, Dr.**
**Mühlenweg 14**
**D-7806 March-Hugstetten(DE)**
Erfinder: **Schächtele, Christoph, Dr.**
**Spechtweg 31**
**D-7800 Freiburg(DE)**
Erfinder: **Mannhardt, Karl, Dr.**
**Graf-Toerringstrasse 2**
**D-8031 Steinebach a. Wörthsee(DE)**
Erfinder: **Rudolph, Claus, Dr.**
**Riedmattenstrasse 11**
**D-7801 Vörstetten(DE)**
Erfinder: **Marmé, Dieter, Prof. Dr.**
**Kaschnitzweg 25**
**D-7800 Freiburg(DE)**

(54) **2-Propylamin-Derivate, Verfahren zu deren Herstellung sowie deren Verwendung.**

(57) Die Erfindung betrifft 2-Propylamin-Derivate der allgemeinen Formel I

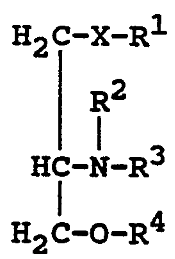

(I),

in welcher

$R^1$ eine geradkettige oder verzweigte, gesättigte oder ungesättigte Alkylgruppe mit bis zu 20 Kohlenstoffatomen darstellt,

$R^2$ und $R^3$ entweder gleich oder verschieden sein können und Wasserstoff oder eine geradkettige oder verzweigte Alkylgruppe mit bis zu 4 Kohlenstoffatomen bedeuten, wobei das zugrunde liegende Stickstoffatom auch mit einem Alkylhalogenid quaternisiert sein kann,

$R^4$ Wasserstoff oder eine Acyl-oder Aroylgruppe mit bis zu 12 Kohlenstoffatomen darstellt und

X Sauerstoff oder Schwefel bedeutet,

sowie gegebenenfalls deren pharmakologisch unbedenkliche Salze, ausgenommen diejenigen Verbindungen, bei denen $R^2$, $R^3$ und $R^4$ gleichzeitig Wasserstoff bedeuten.

EP 0 279 458 A1

Die erfindungsgemäßen Verbindungen der allgemeinen Formel I weisen wertvolle pharmakologische Eigenschaften auf. Sie hemmen die Proteinkinase C (PKC) und verhindern die durch Diacylglycerole wie zum Beispiel Oleoylacetylglycerol stimulierte Thrombozytenaggregation.

Sie dienen daher zur Behandlung von Herz-und Gefäßkrankheiten wie Thrombosen, Arteriosklerose, Hypertension, von rheumatischen Entzündungsprozessen, Allergien, Krebs und bestimmten degenerativen Schäden des Zentralnervensystems.

## 2-Propylamin-Derivate, Verfahren zu deren Herstellung sowie deren Verwendung

Die Erfindung betrifft neue 2-Propylamin-Derivate der allgemeinen Formel I

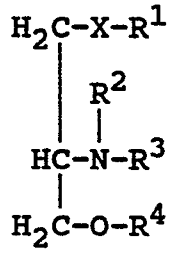

(I),

in welcher

$R^1$ eine geradkettige oder verzweigte, gesättigte oder ungesättigte Alkylgruppe mit bis zu 20 Kohlenstoffatomen darstellt,

$R^2$ und $R^3$ entweder gleich oder verschieden sein können und Wasserstoff oder eine geradkettige oder verzweigte Alkylgruppe mit bis zu 4 Kohlenstoffatom bedeuten, wobei das zugrunde liegende Stickstoffatom auch mit einem Alkylhalogenid mit bis zu 6 C-Atomen quaternisiert sein kann,

$R^4$ Wasserstoff oder eine Acyl-oder Aroylgruppe mit jeweils bis zu 12 Kohlenstoffatomen darstellt und

X Sauerstoff oder Schwefel bedeutet,

sowie gegebenenfalls deren pharmakologisch unbedenkliche Salze, ausgenommen diejenigen Verbindungen, bei denen $R^2$, $R^3$ und $R^4$ gleichzeitig Wasserstoff bedeuten.

Verbindungen der allgemeinen Formel I, bei denen $R^2$, $R^3$ und $R^4$ gleichzeitig Wasserstoff bedeuten, sind bereits beschrieben (vgl. EP 050 148 777) und somit als Stoff nicht Gegenstand dieser Erfindung.

Bevorzugt werden 2-Propylamin-Derivate der allgemeinen Formel I, in welcher

$R^1$ eine Butyl-, Tetradecyl-, Hexadecyl-oder Octadecylgruppe,

$R^2$ und $R^3$ gleich oder verschieden sind und Wasserstoff, Methyl-oder Ethylgruppen bedeuten,

$R^4$ Wasserstoff eine Acetyl-oder Benzoylgruppe darstellt,

X Sauerstoff bedeutet,

ausgenommen diejenigen Verbindungen, bei denen $R^2$, $R^3$ und $R^4$ gleichzeitig Wasserstoff bedeuten.

Besonders bevorzugt wird die Verbindung (±)-2-Ethylamino-3-octadecyloxy-1-propanol.

Die Erfindung betrifft auch Analogie-Verfahren zur Herstellung von 2-Propylamin-Derivaten der allgemeinen Formel I, dadurch gekennzeichnet, daß man

a) entweder in an sich bekannter Weise Verbindungen der allgemeinen Formel II

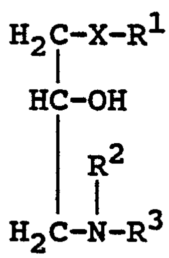

(II),

in der $R^1$, $R^2$, $R^3$ und X die oben angegebene Bedeutung besitzen, zunächst mit anorganischen Säurechloriden in Verbindungen der allgemeinen Formel III

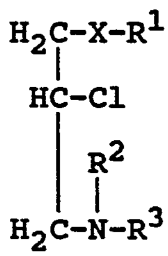

(III),

in der $R^1$, $R^2$, $R^3$ und X wieder die oben angegebene Bedeutung haben, überführt, dies mit Alkoholen der allgemeinen Formel IV

$HO\text{-}CH_2\text{-}R^5$ (IV)

in der $R^5$ einen aromatischen Rest, vorzugsweise den Phenylrest bedeutet, in Gegenwart eines Phasentransferkatalysators zu Verbindungen der allgemeinen Formel V

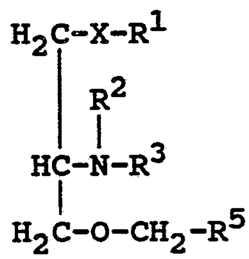

(V),

in welcher $R^1$, $R^2$, $R^3$ und $R^5$ die oben angegebene Bedeutung besitzen, umsetzt, und diese schließlich durch hydrogenolytische Abspaltung der Gruppe $-CH_2\text{-}R^5$ in Verbindungen der allgemeinen Formel I, in denen $R^4$ Wasserstoff bedeutet, überführt, und gewünschtenfalls in bekannter Weise O-acyliert, O-aroyliert und/oder N-alkyliert, oder daß man in an sich bekannter Weise

b) Oxazolin-Derivate der allgemeinen Formel VI

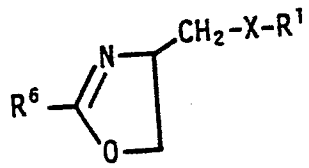

(VI),

in der $R^1$ die oben angegebene Bedeutung hat, $R^6$ einen Arylrest darstellt und X Sauerstoff bedeutet, mit Mineralsäuren zu Verbindungen der allgemeinen Formel I, in denen $R^2$, $R^3$ und $R^4$ Wasserstoff bedeuten, umsetzt und diese gewünschtenfalls anschließend in bekannter Weise O-acyliert, O-aroyliert und/oder N-alkyliert.

Verbindungen der allgemeinen Formel I, bei denen $R^2$ oder $R^3$ für eine Alkylgruppe stehen, werden durch Umsetzung von Verbindungen der allgemeinen Formel I, bei denen $R^2$ und $R^3$ Wasserstoff bedeutet, mit entsprechenden Alkylhalogeniden nach bekannten Verfahren erhalten.

Verbindungen der allgemeinen Formel I, in denen $R^4$ für eine Acyl-oder Aroylgruppe steht, werden hergestellt, indem man entweder Verbindungen der allgemeinen Formel I, in denen $R^4$ Wasserstoff bedeutet, mit entsprechenden Carbonsäureanhydriden nach bekannten Verfahren zur Reaktion bringt, oder Oxazoline der allgemeinen Formel VI, in denen $R^1$ und $R^6$ die oben angegebene Bedeutung besitzen, zunächst mit verdünnter Mineralsäure umsetzt und anschließend gegebenenfalls mit wäßriger Formalinlösung unter reduzierenden Bedingungen an der Aminogruppe alkyliert.

Arylreste bzw. Aroylreste im Sinne der Erfindung sind Reste, deren aromatischer Ring bevorzugt einen Phenyl-, Thienyl, Pyridyl-oder Naphtylrest bedeutet. Besonders bevorzugt ist in beiden Fällen der Phenylrest. Bei einem Aroylrest ist der aromatische Ring mit einer

$-\overset{O}{\overset{\|}{C}}$-Gruppe verbunden.

Verbindungen der allgemeinen Formel I, in denen X ein Schwefelatom darstellt, werden hergestellt, indem man Thiazolidincarbonsäureester der allgemeneinen Formel VII

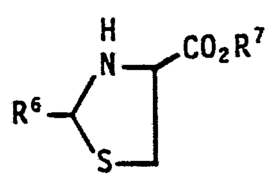

(VII),

in denen $R^6$ die oben angegebene Bedeutung besitzt und $R^7$ eine geradkettige Alkylgruppe mit bis zu 4 Kohlenstoffatomen darstellt, mit Lithiumaluminiumhydrid zu Alkoholen der allgemeinen Formel VIII

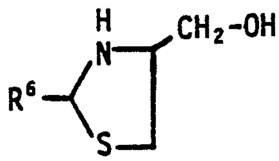

(VIII),

in der $R^6$ die oben angegebene Bedeutung besitzt, reduziert und diese mit Verbindungen der allgemeinen Formel IX

$R^1$-Y (IX)

in denen $R^1$ die oben angegebene Bedeutung hat und Y eine gute Abgangsgruppe darstellt, in Gegenwart eines Alkalimetallhydrides, wie z.B. Natriumhydrid, in einem inerten aprotischen Lösungsmittel, wie z.B. Tetrahydrofuran, Ether oder Dimethylformamid, zur Reaktion bringt und anschließend nach saurer Aufarbeitung in üblicher Weise isoliert und gewünschtenfalls nach an sich bekannten Methoden entweder N-oder O-alkyliert, N-acyliert und/oder aroyliert. Die Herstellung schwefelhaltiger Derivate der allgemeinen Formel I auf diesem Weg ist überraschend und neu, da a priori nicht mit einer Alkylierung am Schwefel zu rechnen war. Als typische Abgangsgruppe eignen sich z.B. Halogenide und Sulfonsäureester.

Die Verbindungen der allgemeinen Formel II sind bekannt (DE-OS 36 25 738). Die Oxazoline der allgemeinen Struktur VI, in denen $R^1$ Octadecyl und $R^5$ Phenyl bedeuten, sind ebenfalls beschrieben (vgl. Tetrahedron Lett. 23, S. 1043, 1982).

Die Thiazolidincarbonsäureester der allgemeinen Formel VIII werden nach bekannten Verfahren durch Umsetzung von Aldehyden der allgemeinen Formel X,

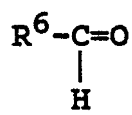

(X)

in denen $R^6$ die oben angegebene Beteutung besitzt, mit Cysteinesterhydrochloriden der allgemeinen Formel XI,

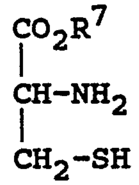

· HCl (XI)

in denen $R^7$ die oben angegebene Bedeutung besitzt, hergestellt (vgl. Liebigs Ann. Chem. 1985 (4), 657).

Quartärsalze am Stickstoffatom der Verbindungen der allgemeinen Formel I werden hergestellt, indem man Verbindungen der allgemeinen Formel I mit Alkyljodiden der allgemeinen Formel XII

$R^8$ -J (XII),

in der $R^8$ eine Methyl-oder Ethylgruppe darstellt, in an sich bekannter Weise umsetzt.

Die Umsetzung der Verbindungen der allgemeinen Formel II zu Verbindungen der allgemeinen Formel III erfolgt in allgemein bekannter Weise mit molaren Mengen oder einem Überschuß, bevorzugt jedoch mit der dreifachen Menge an anorganischen Säurechloriden wie Phosphortrichlorid oder Phosphoroxytrichlorid, bevorzugt jedoch Thionylchlorid, entweder ohne Lösungsmittel oder in einem inerten Lösemittel wie Dichlormethan oder Chloroform bei Temperaturen zwischen 0°C und 80°C, vorzugsweise bei 60°C. Die Reaktionszeit liegt bei 1-12h, vorzugsweise jedoch bei 4h. Die Isolierung der Produkte erfolgt durch Chromatographie und/oder Kristallisation.

Zur Darstellung der 2-Amino-Verbindungen der allgemeinen Formel V werden die Verbindungen der allgemeinen Formel III mit aromatisch substituierten Alkoholen der allgemeinen Formel IV, bevorzugt mit Benzylalkohol, in einem Zweiphasensystem, das aus 50%iger Natronlauge und einem inerten organischen Lösungsmittel, vorzugsweise Dichlormethan, besteht, in Gegenwart äquivalenter Mengen eines Phasentransferkatalysators, wie z.B. Hexadecyltrimethylammoniumchlorid, umgesetzt. Es ist zweckmäßig, bei erhöhter Temperatur, vorzugsweise beim Siedepunkt des organischen Lösungsmittels zu arbeiten. Die

Reaktionszeiten betragen 12-48h, vorzugsweise 24h. Die Reaktionsprodukte lassen sich mit Hilfe bekannter Trennverfahren wie Kristallisation und/oder Chromatographie reinigen und isolieren.

Die hydrogenolytische Abspaltung der Benzylgruppe aus den Verbindungen allgemeinen Formel V erfolgt entweder a) mit Wasserstoff in polaren Lösungsmitteln wie Essigsäure oder Methanol, vorzugsweise Ethanol, bei Atmosphärendruck und Raumtemperatur in Gegenwart von Pd/C als Katalysator oder b) mit Hydraziniumhydroxid in niederen Alkoholen, vorzugsweise in Methanol, beim Siedepunkt des Lösungsmittels in Gegenwart von Pd/C als Katalysator. Die Reindarstellung der Produkte erfolgt durch Kristallisation entsprechender Salze, vorzugsweise der Hydrochloride.

Zur. Herstellung der erfindungsgemäßen N-Alkyl-substituierten Verbindungen der allgemeinen Formel I werden solche Verbindungen bei denen $R^2$ und $R^3$ Wasserstoff bedeuten, mit einem entsprechenden Alkylhalogenid, bevorzugt einem Alkylbromid mit bis zu 4 C-Atomen in einem organischen Lösungsmittel wie Toluol oder auch polaren Solventien wie z. B. Methanol oder Ethanol, vorzugsweise jedoch in Dimethylformamid, bei Temperaturen zwischen 0°C und 100°C, bevorzugt zwischen 40 und 60°C, umgesetzt. Die Reaktionszeiten bewegen sich zwischen 1 und 10h, vorzugsweise liegen sie bei 4h. Die Isolierung und Reinigung der Produkte erfolgt durch Chromatographie und/oder Kristallisation.

Die O-Acylierung der Aminoalkohole der allgemeinen Formel I erfolgt entweder nach Variante a), indem man nach bekanntem Verfahren (vgl. Houben-Weyl, Bd. VIII, S. 547 ff) eine äquivalente oder überschüssige, bevorzugt jedoch die doppelte Menge eines entsprechenden Carbonsäureanhydrides zusammen mit einem tert. Amin, bevorzugt wasserfreiem Pyridin, als Hilfsbase und Lösungsmittel zugleich, mit dem betreffenden Aminoalkohol im Temperaturbereich von 5-50°C, vorzugsweise jedoch bei 25°C, für 15-30h, bevorzugt für 24h, zur Reaktion bringt, oder nach Variante b), indem man, wiederum nach bekanntem Verfahren (vgl. Houben-Weyl, Bd. VIII, S. 543 ff) eine äquivalente Menge eines entsprechenden Carbonsäurechlorides zusammen mit der äquivalenten Menge eines tert. Amins, bevorzugt Triethylamin, in einem inerten Lösungsmittel wie Dichlormethan, 1,1,2-Trichlorethan oder auch Toluol mit dem betreffenden Aminoalkohol bei Temperaturen von 5-50°C, bevorzugt 25°C für 1-4h, vorzugsweise jedoch 2h, reagieren läßt. Die Isolierung und Reinigung der Produkte erfolgt für beide Varianten durch Chromatographie und/oder Kristallisation.

Die Umsetzung der Oxazolin-Derivate der allgemeinen Formel VI zu den erfindungsgemäßen Verbindungen der allgemeinen Formel I, in denen $R^2$ und $R^3$ Wasserstoff bedeuten, erfolgt durch Umsetzung mit verdünnter Mineralsäure wie Schwefelsäure oder Bromwasserstoffsäure, bevorzugt jedoch Salzsäure, bei Temperaturen zwischen 20 und 80°C, bevorzugt jedoch bei 40-60°C. Die Reaktionszeit beträgt 4-24h, vorzugsweise jedoch 8-14h. Die Zugabe organischer Lösungsmittel wie Tetrahydrofuran kann hilfreich sein.

Zur Alkylierung der Aminogruppe wird mit wäßriger Formalinlösung nach Zusatz von niederen Alkoholen wie Methanol, Ethanol oder Isopropylalkohol bevorzugt bei Atmosphärendruck und Raumtemperatur in Gegenwartvon Pd/C als Katalysator mit Wasserstoff umgesetzt.

Die Herstellung der Thiazolidin-Derivate der allgemeinen Formel VII erfolgt nach bekanntem Verfahren, indem man Cysteinester der allgemeinen Formel XI mit einem Überschuß Aldehyd der allgemeinen Formel X in polaren Lösungsmitteln, bevorzugt in einem Gemisch aus Wasser und Ethanol, bei Temperaturen zwischen 60 und 100°C, vorzugsweise bei 80°C für 30 Minuten bis 2h, bevorzugt für 1h, zur Reaktion bringt. Überschüssiger Aldehyd wird entweder destillativ oder als Bisulfit-Addukt entfernt.

Die so dargestellten Thiazolidincarbonsäureester werden in die entsprechenden Alkohole der allgemeinen Struktur VIII übergeführt, indem man die entsprechenden Ester in einem aprotischen Lösungsmittel, vorzugsweise in Diethylether, bei Temperaturen zwischen 0 und 30°C, vorzugsweise bei 10°C, mit Lithiumaluminiumhydrid versetzt. Die Aufarbeitung des Reaktionsgemisches erfolgt unter basischen Bedingungen, vorzugsweise durch Zugabe von 2N Natronlauge. Die Reinigung der Produkte erfolgt durch Chromatographie und/oder Kristallisation. Die erfindungsgemäßen 2-Amino-3-octadecylthio-1-propanole der allgemeinen Formel I werden hergestellt, in dem man die 4-Hydroxymethylthiazolidine der allgemeinen Struktur VIII in aprotischen Lösungsmitteln, vorzugsweise in Tetrahydrofuran, in die Natriumsalze überführt und anschließend mit Verbindungen der allgemeinen Formel IX im selben Lösungsmittel bei Temperaturen zwischen 40 und 100°C, vorzugsweise bei 70°C, für 6-24h, vorzugsweise bei 8-12h, umsetzt. Die Reindarstellung der Produkte erfolgt wieder durch Chromatographie und/oder Kristallisation.

Zur Herstellung von Quartärsalzen werden Verbindungen der allgemeinen Struktur I mit einem entsprechenden Quaternisierungsmittel der allgemeinen Struktur XII in einem polaren Lösungsmittel wie Acetonitril oder einem Alkohol, bevorzugt jedoch Nitromethan bei Temperaturen zwischen 50 und 150°C, bevorzugt jedoch 80-100°C für 1-10h, vorzugsweise jedoch 4-6h, umgesetzt. Die Isolierung und Reinigung der Produkte erfolgt durch Chromatographie und/oder Kristallisation.

Da die erfindungsgemäßen Verbindungen der allgemeinen Formel I an C-2 ein chirales Zentrum aufweisen können, liegen sie entweder als racemische Gemische oder in Form der Enantiomeren vor.

Da die Verbindungen der allgemeinen Formel I an C-2 ein basisches Zentrum besitzen, überführt man sie zum Zwecke der Reinigung und aus galenischen Gründen mit Hilfe von anorganischen und organischen Säuren bevorzugt in kristalline, pharmakologisch verträgliche Salze. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Oxalsäure oder Bernsteinsäure in Frage.

Die erfindungsgemäßen Verbindungen der allgemeinen Formel I weisen wertvolle pharmakologische Eigenschaften auf. Sie hemmen die Proteinkinase C (PKC) und verhindern die durch Diacylglycerole wie zum Beispiel Oleoylacetylglycerol stimulierte Thrombozytenaggregation.

Die Proteinkinase C spielt eine herausragende Rolle bei der Regulation zellulärer Vorgänge, die eng verknüpft sind mit der physiologischen Kontrolle von kontraktilen, sekretorischen und proliferativen Prozessen (Y. Nishizuka, Nature 308, S. 593-698 (1984)). Die physiologische Aktivierung der Proteinkinase C erfolgt über einen durch rezeptor-vermittelten Abbau membrangebundener Phosphatidylinositole entstandenen "second messenger", das Diacylglycerol.

Aufgrund dieser Eigenschaften können die erfindungsgemäßen Verbindungen zur Behandlung von Herz-und Gefäßkrankheiten wie Thrombosen, Arteriosklerose, Hypertension, von rheumatischen Entzündungsprozessen, Allergien, Krebs und bestimmten degenerativen Schäden des Zentralnervensystems verwendet werden.

Die erfindungsmäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat-und Citrat-Puffer, Ethanol, Komplexbildner (wie Ethylendiamintetraessigsäure und deren nichttoxische Salze) sowie hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol); für orale Applikation geeignete Zubereitungen können gewünschtenfalls zusätzliche Geschmacks-und/oder Süßstoffe enthalten.

Die verabreichten Einzeldosen liegen im Bereich von etwa 1 bis 1000 mg, vorzugsweise 20 - 500 mg.

Die folgenden Beispiele sollen die Erfindung näher erläutern:

## BEISPIEL 1

(±)-2-Dimethylamino-3-octadecyloxy-1-propylacetat • Hydrochlorid

130 mg (0,35 mmol) (±)-2-Dimethylamino-3-octadecyloxy-1-propanol • Hydrochlorid werden mit 120 mg (1 mmol) Acetanhydrid in 5 ml wasserfreiem Pyridin 48h bei Raumtemperatur gerührt. Man destilliert anschließend das Lösungsmittel im Vakuum ab, nimmt den Rückstand in 20 ml n-Pentan auf und isoliert das feste Produkt nach Kühlung der Lösung auf -20°C, Schmp. 38-45°C. Ausbeute 54,5%.

Das als Vorstufe verwendete (±)-2-Dimethylamino-3-octadecyloxy-1-propanol • Hydrochlorid wird wie folgt hergestellt:

230 (0,5 mmol) (±)-3-Benzyloxy-2-dimethylamino-1-octadecyloxypropan werden in 15 ml Methanol gelöst, mit 750 mg Pd/C (10%ig) und 0,5 ml Hydrazinhydrat versetzt und sodann 4h am Rückfluß erhitzt. Man läßt abkühlen, filtriert die Reaktionslösung, wäscht mit warmem Methanol nach und destilliert das Lösungsmittel im Vakuum ab. Den Rückstand nimmt man in 100 ml Dichlormethan auf, versetzt ihn mit 20 ml 2N HCl und rührt 15 Minuten kräftig bei Raumtemperatur. Anschließend wird die organische Phase abgetrennt, getrocknet und im Vakuum vom Lösungsmittel betreit. Das freie Produkt wird ohne weitere Reinigung in die nächste Stufe eingesetzt Schmp. 48-53°C.

Das als Vorstufe verwendete (±)-3-Benzyloxy-2-dimethylamino-1-octadecyloxypropan wird wie folgt hergestellt:

1,0 g (2, 6 mmol) (±)-2-Chlor-3-dimethylamino-1-octadecyloxypropan, 0,28 g (2,6 mmol) Benzylalkohol, 2,0 ml 50%ige Natronlauge und 3,5 g (2,6 mmol) einer 25%igen Lösung von Hexadecyltrimethylammoniumchlorid werden in 50 ml Dichlormethan 24h unter Rückfluß erhitzt. Zur abgekühlten Reaktionslösung gibt man 100 ml Wasser und 100 ml Dichlormethan, separiert die organische Phase, extrahiert die wäßrige Phase noch einmal mit 50 ml Dichlormethan und trocknet die vereinigten organischen Extrakte über Natriumsulfat. Nach dem Abdestillieren des Lösungsmittels im Vakuum wird der Rückstand säulenchromatographisch aufgetrennt (Kieselgel, Dichlormethan/Methanol = 18:1). Mit der Fraktion wird das Produkt isoliert und in die nächste Stufe eingesetzt Schmp. 15°C.

Das als Vorstufe verwendete (±)-2-Chlor-3-dimethylamino-1-octadecyloxypropan • Hydrochlorid wird wie folgt hergestellt:

In eine Lösung von 6,5 g (16 mmol) (±)-3-Dimethylamino-1-octadecyloxy-2-propanol in 60 ml Chloroform werden bei Raumtemperatur 5,7 g (48 mmol) Thionylchlorid eingetropft und anschließend 4h unter Rückfluß erhitzt. Zum abgekühlten Reaktionsgemisch gibt man 100 ml Wasser und 100 ml Chloroform, separiert anschließend die organische Phase und wäscht sie mit 30 ml 10%iger Natriumhydrogencarbonatlösung. Nach dem Trocknen der organischen Phase über Natriumsulfat wird diese im Vakuum vom Lösungsmittel befreit und säulenchromatographisch aufgetrennt (Kieselgel, Dichlormethan/ Methanol = 9:1). Weißer Feststoff vom Schmp. 65-85°C.

In analoger Weise werden hergestellt:

(±)-2-Dimethylamino-3-hexadecyloxy-1-propylacetat Oxalat (1.a) Schmp. 112-117°C aus Ethylacetat, Ausbeute: 14,5%.

(±)-2-Dimethylamino-3-tetradecyloxy-1-propylacetat Oxalat (1.b) Schmp. 108-115°C aus n-Pentan, Ausbeute: 77,8%.

(±)-2-Dimethylamino-3-butoxy-1-propylacetat Oxalat (1.c) Schmp. 111°C aus Methanol/Ether, Ausbeute: 100%.

(±)-2-Diethylamino-3-octadecyloxy-1-propylacetat Hydrochlorid (1.d)
Schmp. 42-52°C aus n-Pentan, Ausbeute: 37,6%.

## BEISPIEL 2

(±)-2-Ethylamino-octadecyloxy-1-propanol • Hydrochlorid

570 mg (1,7 mmol) (±)-2-Amino-3-octadecyloxy-1-propanol (vgl. Tetrahedron Lett. 23, 1043, 1982) werden in 30 ml wasserfreiem Dimethylformamid aufgenommen, mit 1,23 g (11 mmol) Ethylbromid versetzt und 4h auf 40°C erwärmt. Man destilliert sodann das Lösungsmittel im Vakuum ab, nimmt den Rückstand in 100 ml Dichlormethan auf und wäscht ihn mit Wasser. Nach Freisetzen der Base mit 2N Natronlauge wird die organische Phase über $Na_2SO_4$ getrocknet, das Produkt als Hydrochlorid gefällt und anschließend chromatographisch gereinigt. (Kieselgel, Dichlormethan/ Methanol = 9:1). Farbloses Produkt vom Schmp. 72-78°C, Ausbeute 29,4%.

In analoger Weise wurde hergestellt:

(±)-2-Diethylamino-3-octadecyloxy-1-propanol Oxalat (2.a). Schmp. 43-48°C aus n-Pentan, Ausbeute: 4,4 %.

## BEISPIEL 3

(±)-2-Dimethylamino-3-octadecyloxy-1-propylbenzoat

6,0 g (13,4 mmol) (±)-2-Amino-3-octadecyloxy-1-propylbenzoat werden mit 4,35 g (53,7 mmol) 37%iger Formalinlösung in 400 ml Ethanol in Gegenwart von 2,5 g Pd/C als Katalysator bei Atmosphärendruck und Raumtemperatur bis zur Sättigung mit Wasserstoff umgesetzt. Man filtriert anschließend, destilliert das Lösungsmittel im Vakuum ab und trennt den Rückstand säulenchromatographisch auf (Kieselgel, Dichlormethan/Methanol = 27:1). Hellgelbes, viskoses Öl, Ausbeute 26,0%.

$^1$H-NMR ($CDCl_3$): 8.15-7.9 (2H,m), 7.5-7.2 (3H,m), 4.5-4.3 (2H,m), 3.6-3.5 (2H,m), 3.40 (2H,t), 2.95 (1H,m), 2.40 (6H,s), 1.7-1.0 (32H,m), 0.80 (3H.t).

Das als Vorstufe verwendete (±)-2-Amino-3-octadecyloxy-1-propylbenzoat wird wie folgt hergestellt:

5,56 g (13 mmol) (±)-4-Octadecyloxymethyl-2-phenyl-2-oxazolin (vgl. Tetrahedron Lett. 23, 1043, 1982) werden in 150 ml Tetrahydrofuran gelöst, mit 130 ml (13 mmol) 0,1N Salzsäure versetzt und 24h bei 40°C gerührt. Man destilliert anschließend die Lösungsmittel im Vakuum ab, setzt mit 2N Natronlauge die Base frei und extrahiert das Produkt mit Dichlormethan. Nach dem Trocknen der organischen Phase über Natriumsulfat und Abdestillieren des Lösungsmittels im Vakuum wird das Produkt als farbloses Öl isoliert.

## BEISPIEL 4

(±)-1-Benzoyloxy-3-octadecyloxy-2-propyl-trimethylammoniumjodid

720 mg (1,5 mmol) (±)-2-Dimethylamino-3-octadecyloxy-1-propylbenzoat werden in 30 ml Nitromethan mit 650 mg (4,5 mmol) Jodmethan versetzt und für 3h auf 80°C erwärmt. Nach dem Abkühlen wird der Niederschlag abgesaugt und die Mutterlauge zur Trockne eingeengt. Niederschlag und Rückstand kristallisiert man aus Essigester um und isoliert das (±)-1-Benzoyloxy-3-octadecyloxy-2-propyl-trimethylammoniumjodid als hellgelbes Produkt vom Schmp. von 100-102°C, Ausbeute 53,5%.

BEISPIEL 5

(±)-2-Amino-3-octadecylthio-1-propanol

In eine bei Raumtemperatur unter Stickstoffatmosphäre gerührte Suspension aus 0,9 g (30 mmol) 80%igem Natriumhydrid in Paraffinöl und 60 ml wasserfreiem Tetrahydrofuran werden langsam 5,5 g (30 mmol) 4-Hydroxymethyl-2-phenyl-thiazolidin, gelöst in 100 ml wasserfreiem Tetrahydrofuran, getropft. Nach 1h läßt man eine Lösung von 9,8 g (30 mmol) Octadecylmesylat in 150 ml Tetrahydrofuran einfließen und erwärmt die Reaktionslösung auf 80°C. Nach 8h Rühren bei dieser Temperatur läßt man erkalten, destilliert anschließend das Lösungsmittel im Vakuum ab und nimmt den Rückstand in 300 ml Dichlormethan auf. Die organische Phase wird gewaschen, getrocknet und bis zur sauren Reaktion mit einer bei Raumtemperatur gesättigten Lösung von HCl in Ether versetzt. Man destilliert die Lösungsmittel im Vakuum ab und fraktioniert den Rückstand säulenchromatographisch (Kieselgel, Dichlormethan/Methanol = 9:1). Das mit der dritten Fraktion isolierte Produkt wird aus Ether umkristallisiert. Hellbeige Kristalle vom Schmp. 79-81°C, Ausbeute 19,0%.

Das als Ausgangsmaterial verwendete 4-Hydroxymethyl-2-phenylthiazolidin wird wie folgt hergestellt:

Eine Lösung von 10,0 g (40 mmol) 2-Phenyl-4-thiazolidincarbonsäureethylester in 200 ml wasserfreiem Ether wird bei 0°C und unter Stickstoffatmosphäre langsam mit 0,8 g (20 mmol) Lithiumaluminiumhydrid versetzt. Man läßt auf Raumtemperatur erwärmen, rührt noch 3h und fügt sodann bis pH 9 2N Natronlauge hinzu. Man verdünnt mit 100 ml Wasser, trennt die organische Phase ab und extrahiert die wäßrige Phase noch zweimal mit je 50 ml Ether. Nach dem Trocknen der vereinigten organischen Extrakte über Natriumsulfat und Abdestillieren des Lösungsmittels im Vakuum wird der Rückstand säulenchromatographisch aufgetrennt (Kieselgel, Dichlormethan/Methanol = 18:1). Man isoliert das Produkt in Form eines hellgelben Öls.

Der als Ausgangsmaterial verwendete 2-Phenyl-4-thiazolidincarbonsäureethylester wird wie folgt hergestellt:

Eine Lösung von 31,0 g (0,17 mol) Cysteinethylester 12A Hydrochlorid in 200 ml Wasser wird mit 85 ml (0,17 mol) 2N Natronlauge versetzt, auf 50°C erwärmt und sodann mit einer Lösung von 39,4 g (0,37 mol) Benzaldehyd in 60 ml Ethanol versetzt. Man erwärmt 30 Minuten auf 80°C, läßt auf Raumtemperatur abkühlen und trennt die ölige organische Phase ab. Diese wird in 300 ml Pentan aufgenommen, eine halbe Stunde bei Raumtemperatur kräftig gerührt und aufgetrennt. Die Pentanphase wird verworfen; die zweite Phase nimmt man in 100 ml Ether auf und trennt den restlichen Benzaldehyd als Bisulfit-Addukt ab. Nach Abdestillieren des Lösungsmittels im Vakuum wird der Thiazolidincarbonsäureethylester als hellgelbes Öl isoliert.

Die folgenden Vergleichsversuche veranschaulichen die pharmakologische Wirksamkeit der erfindungsgemäßen Verbindungen der allgemeinen Formel I:

1. Hemmung von PKC-Aktivatoren abhängiger und PKC-Aktivatoren unabhängiger Kinase-Aktivität.

Zur Klärung des hemmenden Einflusses der erfindungsgemäßen Verbindungen der allgemeinen Formel I auf die Calcium-und Phospholipid-abhängige Proteinkinase (PKC) wurde diese Enzymaktivität ausgehend von einem Hühnermagenextrakt angereichert. In Anlehnung an in der Literatur beschriebene Reinigungsschritte (J. Biol. Chem. 260, S. 15718-15722, 1985) wurde bei der aus 2 Schritten bestehenden Reinigung die Eigenschaft des Enzyms ausgenutzt, in Anwesenheit von Calcium an Zellmembranen zu binden und durch Calciumchelatoren (EGTA) wieder abgelöst zu werden. Beim 1. Anreicherungsschritt erfolgte die Bindung der PKC an Membranen des Hühnermagenextraktes und im 2. Schritt an sogenannte Inside/Out-Vesikel von Erythrozyten. Nach Ablösung von den Erythrozyten-Membranen und Umpufferung lag die PKC-Präparation in 10 mM HEPES, 1 mM DTT, 0,1% PEG 20000, pH 7,5 vor und konnte unter diesen

Bedingungen bei -70°C für mehrere Monate ohne Aktivitätsverlust gelagert werden.

Die Aktivität des Enzyms wurde über den Einbau von Phosphor-32-markiertem Phosphat in das durch PKC phosphorylierbare Protein Histon H-1 bestimmt. Der Test enthält dabei folgende Kumponenten: 50 mM HEPES (pH 7,5), 1 mM DTT, 5 mM $MgCl_2$, ca. 10 µM freies $Ca^{2+}$, 200 µg/ml Histon, 5 µg/ml Phosphatidylserin, 1 µg/ml 1,2-Diolein, 10 µM ATP, eine jeweils geeignete Menge der PKC-Präparation und gegebenenfalls eine Testsubstanz. Die unter Standardbedingungen gemessene Kinase-Aktivität in Anwesenheit von PKC-Aktivatoren (und ohne Testsubstanz) wurde jeweils als 100% gesetzt und die Hemmwirkung der Verbindungen der allgemeinen Formel I prozentual darauf bezogen.

In Tabelle I ist die Inhibierung von PS/DO [PS: Phosphatidylserin, DO: 1,2-Diolein] vermittelter Proteinkinase C-Aktivität angegeben.

2. Oleoylacetylglycerol (OAG)-induzierte Plättchenaggregation

a) Beschreibung des experimentellen Ablaufs

Für die Durchführung der Aggregationsexperimente werden gelfiltrierte Human-Thrombozyten verwendet, die folgendermaßen bereitgestellt werden:

Nach Zentrifugation von Humanblut für 15 Minuten bei 60 × g wird der Überstand, das plättchenreiche Plasma, einer Gelfiltration unterzogen. Durch die Gelfiltration mittels einer mit Sepharose CL-2B gepackten Säule werden die Plättchen in ein calciumfreies, definiertes Puffermilieu (15 mM Tris, 150 mM NaCl, 5,5 mM Glucose, 0,2% Rinderserum-Albumin, pH 7,4) überführt. Anschließend werden die Plättchen auf eine Konzentration von $7 \times 10^{-4}$ M Calcium recalcifiziert und dann bei 37°C in einem Aggregometer inkubiert. Nach Auslösung der Aggregation durch den Stimulus wird die Abnahme der Absorption als Maß für die Aggregation bestimmt. Als aggregationsauslösender Stimulus wird 1-Oleoyl-2-acetylglycerol (OAG), ein Aktivator des Enzyms Proteinkinase C (J. Biol. Chem. 258, S. 6701-6704, 1983) in einer Konzentration von $5 \times 10^{-5}$ M verwendet. Die unter diesen Bedingungen erzielte Aggregation wird als 100%-Kontrolle betrachtet.

Der Einfluß einer Substanz auf die OAG-induzierte Aggregation wird derart bestimmt, daß die Plättchen zuerst 3 Minuten mit der jeweiligen Konzentration der Testsubstanz inkubiert wurden und anschließend mit OAG die Aggregation ausgelöst wird. Das reduzierte Ausmaß der Aggregation wird jeweils als Prozentsatz der 100%-Kontrolle berechnet und daraus der Prozentsatz der Inhibierung bestimmt.

Die Inhibierung der OAG-induzierten Plättchenaggregation ist ebenfalls in Tabelle I dargestellt.

Tabelle 1

| Beispiel | konz. (mol/l) | Inhibierung der PS/DO-vermittelten PKC-Aktivität (%) | Inhibierung der OAG-induzierten Plättchen Aggregation (%) |
|---|---|---|---|
| 1 | $1x10^{-4}$ | | 99 |
| | $3x10^{-5}$ | | 69 |
| | $1x10^{-5}$ | 28 | 32 |
| 1a | $1x10^{-4}$ | | 100 |
| | $3x10^{-5}$ | | 23 |
| | $1x10^{-5}$ | 23 | 0 |
| 1b | $3x10^{-5}$ | | 100 |
| | $1x10^{-5}$ | 36 | 59 |
| 2 | $1x10^{-4}$ | 99 | |
| | $1x10^{-5}$ | 91 | 69 |
| | $3x10^{-6}$ | | 14 |
| | $1x10^{-6}$ | 23 | |
| 2a | $1x10^{-4}$ | 100 | |
| | $1x10^{-5}$ | 78 | 82 |
| | $3x10^{-6}$ | | 21 |
| | $1x10^{-6}$ | 11 | 2 |
| 4 | $1x10^{-4}$ | 104 | |
| | $3x10^{-5}$ | | 99 |
| | $1x10^{-5}$ | 90 | 28 |
| | $1x10^{-6}$ | 21 | 6 |
| 5 | $1x10^{-5}$ | 39 | 35 |
| | $1x10^{-6}$ | 12 | 0 |
| 6 | $1x10^{-5}$ | 69 | 82 |
| | $3x10^{-6}$ | | 21 |
| | $1x10^{-6}$ | 11 | 2 |

Verbindung 6 = (±)-2-Amino-3-octadecyloxy-1-propanol
[Tetrahydron Letters 23 S. 1043 (1982)]

**Ansprüche**

1.) 2-Propylamin-Derivative der allgemeinen Formel I

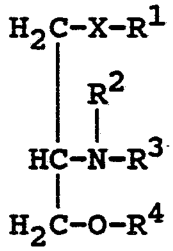

(I),

in welcher

$R^1$ eine geradkettige oder verzweigte, gesättigte oder ungesättigte Alkylgruppe mit bis zu 20 Kohlenstoffatomen darstellt,

$R^2$ und $R^3$ entweder gleich oder verschieden sein können und Wasserstoff oder eine geradkettige oder verzweigte Alkylgruppe mit bis zu 4 Kohlenstoffatomen bedeuten, wobei das zugrunde liegende Stickstoffatom auch mit einem Alkylhalogenid mit bis zu 6 C-Atomen quaternisiert sein kann,

$R^4$ Wasserstoff oder eine Acyl-oder Aroylgruppe mit bis zu 12 Kohlenstoffatomen darstellt und

X Sauerstoff oder Schwefel bedeutet,

sowie gegebenenfalls deren pharmakologisch unbedenklichen Salze, ausgenommen diejenigen Verbindungen, bei denen $R^2$, $R^3$ und $R^4$ gleichzeitig Wasserstoff bedeuten.

2.) 2-Propylamin-Derivate nach Anspruch 1 der allgemeinen Formel I, in welcher

$R^1$ eine Butyl-, Tetradecyl-, Hexadecyl-oder Octadecylgruppe,

$R^2$ und $R^3$ gleich oder verschieden sind und Wasserstoff, Methyl-oder Ethylgruppen bedeuten,

$R^4$ Wasserstoff, eine Acetyl-oder Benzoylgruppe darstellt,

X Sauerstoff bedeutet,

sowie deren pharmakologisch unbedenklichen Salze, ausgenommen diejenigen Verbindungen, bei denen $R^2$, $R^3$ und $R^4$ gleichzeitig Wasserstoff bedeuten.

3.) (±)-2-Ethylamino-3-octadecyloxy-1-propanol.

4.) Verfahren zur Herstellung von 2-Propylamin-Derivaten der allgemeinen Formel I nach Anspruch 1 bis 3, dadurch gekennzeichnet, daß man

a) entweder in an sich bekannter Weise Verbindungen der allgemeinen Formel II

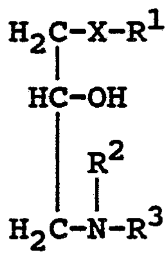

(II),

in der $R^1$, $R^2$, $R^3$ und X die oben angegebene Bedeutung besitzen, zunächst mit anorganischen Säurechloriden in Verbindungen der allgemeinen Formel III

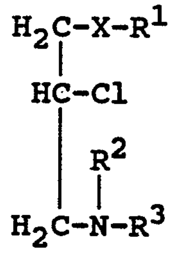

(III),

in der $R^1$, $R^2$, $R^3$ und X wieder die oben angegebene Bedeutung haben, überführt, diese mit Alkoholen der allgemeinen Formel IV

$HO\text{-}CH_2\text{-}R^5$ (IV)

in der $R^5$ einen aromatischen Rest, vorzugsweise den Phenylrest bedeutet, in Gegenwart eines Phasentransferkatalysators zu Verbindungen der allgemeinen Formel V

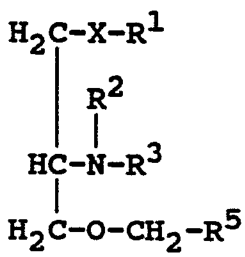

(V),

in welcher $R^1$, $R^2$, $R^3$ und $R^5$ die oben angegebene Bedeutung besitzen, umsetzt, und diese schließlich durch hydrogenolytische Abspaltung der Gruppe $-CH_2\text{-}R^5$ in Verbindungen der allgemeinen Formel I, in denen $R^4$ Wasserstoff bedeutet, überführt, und gewünschtenfalls in bekannter Weise O-acyliert, O-aroyliert und/oder N-alkyliert, oder daß man in an sich bekannter Weise

b) Oxazolin-Derivate der allgemeinen Formel VI

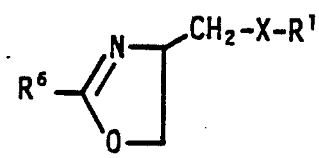

(VI),

in der $R^1$ die oben angegebene Bedeutung hat, $R^6$ einen Arylrest darstellt und X Sauerstoff bedeutet mit Mineralsäuren zu Verbindung der allgemeinen Formel I, in denen $R^2$, $R^3$ und $R^4$ Wasserstoff bedeuten, umsetzt und diese gewünschtenfalls anschließend in bekannter Weise O-acyliert, O-aroyliert und/oder N-alkyliert und in pharmakologisch unbedenkliche Salze überführt.

5.) Arzneimittel enthaltend mindestens eines der 2-Propylamin-Derivate der allgemeinen Formel I gemäß Anspruch 1
sowie gegebenenfalls deren pharmakologisch unbedenklichen Salze als Wirkstoff.

6.) Verwendung von 2-Propylamin-Derivaten der allgemeinen Formel I gemäß Anspruch 1
sowie gegebenenfalls deren pharmakologisch unbedenklichen Salze zur Herstellung von Arzneimitteln zur Bekämpfung von Herz-und Gefäßkrankheiten, rheumatischen Entzündungsprozessen, Allergien, Krebs und bestimmten degenerativen Schäden des Zentralnervensystems.

Patentansprüche für folgende Vertragsstaaten: ES, GR.

1.) Verfahren zur Herstellung von 2-Propylamin-Derivaten der allgemeinen Formel I

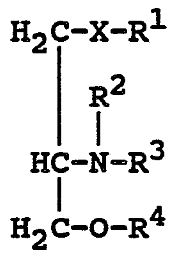

(I),

in welcher

$R^1$ eine geradkettige oder verzweigte, gesättigte oder ungesättigte Alkylgruppe mit bis zu 20 Kohlenstoffatomen darstellt,

$R^2$ und $R^3$ entweder gleich oder verschieden sein können und Wasserstoff oder eine geradkettige oder verzweigte Alkylgruppe mit bis zu 4 Kohlenstoffatomen bedeuten, wobei das zugrunde liegende Stickstoffatom auch mit einem Alkylhalogenid mit bis zu 6 C-Atomen quaternisiert sein kann,

$R^4$ Wasserstoff oder eine Acyl-oder Aroylgruppe mit bis zu 12 Kohlenstoffatomen darstellt und

X Sauerstoff oder Schwefel bedeutet,

sowie gegebenenfalls deren pharmakologisch unbedenklichen Salze, ausgenommen diejenigen Verbindungen, bei denen $R^2$, $R^3$ und $R^4$ gleichzeitig Wasserstoff bedeuten, dadurch gekennzeichnet, daß man

a) entweder in an sich bekannter Weise Verbindungen der allgemeinen Formel II

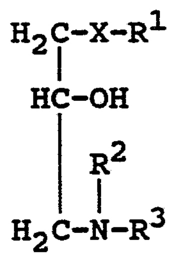

(II),

in der $R^1$, $R^2$, $R^3$ und X die oben angegebene Bedeutung besitzen, zunächst mit anorganischen Säurechloriden in Verbindungen der allgemeinen Formel III

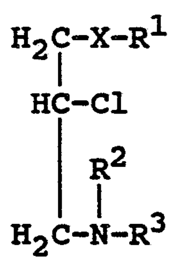

(III),

in der $R^1$, $R^2$, $R^3$ und X wieder die oben angegebene Bedeutung haben, überführt, diese mit Alkoholen der allgemeinen Formel IV

$HO\text{-}CH_2\text{-}R^5$ (IV),

in der $R^5$ einen aromatischen Rest, vorzugsweise den Phenylrest bedeutet, in Gegenwart eines Phasentransferkatalysators zu Verbindungen der allgemeinen Formel V

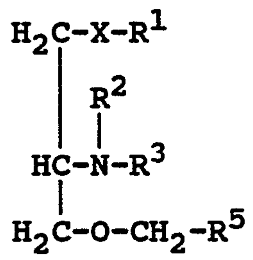

(V),

in welcher $R^1$, $R^2$, $R^3$ und $R^5$ die oben angegebene Bedeutung besitzen, umsetzt, und diese schließlich durch hydrogenolytische Abspaltung der Gruppe $-CH_2-R^5$ in Verbindungen der allgemeinen Formel I, in denen $R^4$ Wasserstoff bedeutet, überführt, und gewünschtenfalls in bekannter Weise O-acyliert, O-aroyliert und/oder N-alkyliert, oder daß man in an sich bekannter Weise

b) Oxazolin-Derivate der allgemeinen Formel VI

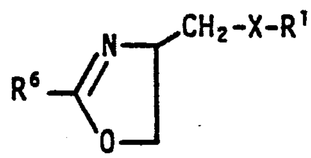

(VI),

in der $R^1$ die oben angegebene Bedeutung hat, $R^6$ einen Arylrest darstellt und X Sauerstoff bedeutet mit Mineralsäuren zu Verbindung der allgemeinen Formel I, in denen $R^2$, $R^3$ und $R^4$ Wasserstoff bedeuten, umsetzt und diese gewünschtenfalls anschließend in bekannter Weise O-acyliert, O-aroyliert und/oder N-alkyliert und in pharmakologisch unbedenkliche Salze überführt.

2.) Verfahren zur Herstellung von 2-Propylamin-Derivaten nach Anspruch 1 der allgemeinen Formel I, in welcher

$R^1$ eine Butyl-, Tetradecyl-, Hexadecyl-oder Octadecylgruppe,

$R^2$ und $R^3$ gleich oder verschieden sind und Wasserstoff, Methyl-oder Ethylgruppen bedeuten,

$R^4$ Wasserstoff, eine Acetyl-oder Benzoylgruppe darstellt,

X Sauerstoff bedeutet,

sowie deren pharmakologisch unbedenklichen Salze, ausgenommen diejenigen Verbindungen, bei denen $R^2$, $R^3$ und $R^4$ gleichzeitig Wasserstoff bedeuten.

3.) Verfahren gemäß Anspruch 1 zur Herstellung von (±)-2-Ethylamino-3-octadecyloxy-1-propanol.

4.) Verwendung von 2-Propylamin-Derivaten der allgemeinen Formel I gemäß Anspruch 1 sowie gegebenenfalls deren pharmakologisch unbedenklichen Salze zur Herstellung von Arzneimitteln zur Bekämpfung von Herz-und Gefäßkrankheiten, rheumatischen Entzündungsprozessen, Allergien, Krebs und bestimmten degenerativen Schäden des Zentralnervensystems.

Europäisches Patentamt

# EUROPÄISCHER RECHERCHENBERICHT

Nummer der Anmeldung

EP 88102467.3

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CHEMICAL ABSTRACTS, Band 104, Nr. 11, 17. März 1986, Columbus, Ohio, USA<br>H. NOMURA et al.: "Glycerol derivatives and their pharmaceutical use" Seite 621, Spalte 2, Zusammenfassung-Nr. 88 143y<br>& EP-A-0 157 609<br>* Chemical Abstracts, Eleventh Collective Index, Formulas Bände 96-105 (1982-1986) Seite 16874F, 2. Spalte, vorletzte Verbindung *<br>-- | 1,2 | C 07 C 93/16<br>C 07 C 93/04<br>C 07 C 149/24<br>A 61 K 31/215 |
| X | EP - B1 - 0 025 083 (EPROVA)<br>* Seite 5, Zeile 49 *<br>-- | 1 | RECHERCHIERTE SACHGEBIETE (Int. Cl.4) |
| X | CHEMICAL ABSTRACTS, Band 105, Nr. 9, 1. September 1986, Columbus, Ohio, USA<br>A.R. DERZHINSKII et al.: "Functional sulfur-containing compounds. 8. Synthesis of sulfur containing aziridines and oxazolidines" Seite 632, Spalte 1, Zusammenfassung-Nr. 78 771h<br>& Izv.Akad.Nauk SSSR, Ser. Khim. 1985,(9), 2090-4<br>* Chemical Abstracts, Eleventh Collective Index, Formulas Bände 96-105(1982-1986), Seite 6468F, $C_{11}H_{25}NOS$(3. Verbindung) *<br>-- | 1 | C 07 C 93/00<br>C 07 C 149/00 |

Der vorliegende Recherchenbericht wurde für alle Patentansprüche erstellt.

| Recherchenort | Abschlußdatum der Recherche | Prüfer |
|---|---|---|
| WIEN | 29-04-1988 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82

Europäisches Patentamt

# EUROPÄISCHER RECHERCHENBERICHT

Nummer der Anmeldung

EP 88102467.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 104, Nr. 19, 12. Mai 1986, Columbus, Ohio, USA<br>A.R. DERZHINSKII et al.: "Functional sulfur-containing compounds. 7. Reactions of 2,3--epoxypropyl alkyl sulfides, sulfoxydes, and sulfones with alcoholates and amines" Seite 623, Spalte 1, Zusammenfassung-Nr. 167 952q<br>* Chemical Abstracts, Eleventh Collective Index, Formulas Bände 96-105 (1982-1986) Seite 6468F, $C_{11}H_{25}NOS$ (2. Verbindung) *<br>-- | 1 | |
| X | CHEMICAL ABSTRACTS, Band 103, Nr. 13, 30. September 1985, Columbus, Ohio, USA<br>J.H. GRIFFIN: "L-Cysteine-, L-methionine- and D-penicillamine derived ligands for transition metal catalyzed carbon-carbon--bond forming reactions" Seite 656, Spalte 1, Zusammenfassung-Nr. 105 278v<br>& J.Org.Chem. 1985, 50(18), 3261-6<br>* Chemical Abstracts, Eleventh Collective Index, Formulas Bände 96-105 (1982-1986) Seite 2564, $C_6H_{15}NOS$ (letzte Verbindung) *<br>-- | 1 | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 29-04-1988 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung



EP 88102467.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| D,A | TETRAHEDRON LETTERS, Band 23, Nr. 10, 1982, Oxford<br>NIZAL S.CHANDRAKUMAR et al. "A new method for the stereospecific synthesis of ether phospholipides. Preparation of the amide analog of platelet-activating factor and related derivatives"<br>Seiten 1043-1046<br>* Seite 1044, Schema I - Verbindung 6 *<br>-- | 1,4 | |
| A | EP - A2 - 0 142 333 (ONO PHARMACEUTICAL)<br>* Zusammenfassung; Ansprüche 1, 10,12,13 *<br>-- | 1,5,6 | |
| A | EP - A1 - 0 053 436 (AMERICAN HOSPITAL SUPLLY)<br>* Ansprüche 1,14-18 *<br>-- | 1,5,6 | RECHERCHIERTE SACHGEBIETE (Int. Cl.4) |
| P,D,A | DE - A1 - 3 625 738 (GÖDECKE)<br>* Zusammenfassung; Ansprüche 1, 6 *<br>---- | 1,5,6 | |

Der vorliegende Recherchenbericht wurde für alle Patentansprüche erstellt.

| Recherchenort | Abschlußdatum der Recherche | Prüfer |
|---|---|---|
| WIEN | 29-04-1988 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument